Europäisches Patentamt

European Patent Office

Office européen des brevets

(11) Publication number: **0 274 762 B1**

(12) ## EUROPEAN PATENT SPECIFICATION

(45) Date of publication of patent specification: **23.10.91**   (51) Int. Cl.5: **A61F 6/06**

(21) Application number: **87200050.0**

(22) Date of filing: **15.01.87**

(54) Pessary.

(43) Date of publication of application:
**20.07.88 Bulletin 88/29**

(45) Publication of the grant of the patent:
**23.10.91 Bulletin 91/43**

(84) Designated Contracting States:
**AT BE CH DE ES FR GB GR IT LI LU NL SE**

(56) References cited:
**DE-A- 1 900 196**
**GB-A- 961 880**
**GB-A- 1 115 727**
**US-A- 4 402 695**
**US-A- 4 516 570**

(73) Proprietor: **FORTUNE CAPITAL MANAGEMENT B.V.**
**Herengracht 182**
**NL-1016 BR Amsterdam(NL)**

(72) Inventor: **Drogendijk, Arie Costiaan**
**Marathon 7**
**NL-2924 XL Krimpen a/d IJssel(NL)**
Inventor: **Kruithof, Cornelis**
**Rododendronstraat 230**
**NL-3053 LR Rotterdam(NL)**

(74) Representative: **van der Beek, George Frans, Ir. et al**
**Nederlandsch Octrooibureau Scheveningseweg 82 P.O. Box 29720**
**NL-2502 LS 's-Gravenhage(NL)**

## Description

The invention relates to a pessary which is particularly applicable in the treatment of prolapse of the internal female sex organs, which pessary is formed by a ring-shaped member comprising a core of a malleable metal encased in a flexible casing of which at the least the outside surface is made of biocompatible material such as a synthetic material, so that said member can be deformed by hand to any given shape, after which the member retains the ultimately given shape.

Prolapses of the internal female sex organs result from laceration of stretching of the supporting connective tissues linking these organs to the pelvic wall. The cause may be attributed to many factors, the most prevalent being hormonal and physical effects of pregnancy and childbirth.

A prolapse may affect various parts of the internal organs, to wit the uterus and the anterior and posterior vaginal walls, taking into account that one as well as several of these parts may be affected. A prolapse of the uterus takes place as a result of weakening of the ligaments which connect it to the pelvic wall. A prolapse of the anterior vaginal wall results from weakening of the supportive tissues located between the bladder and the vagina: the bladder descends and forces the anterior vaginal wall with it.

A prolapse of the posterior vaginal wall may occur in two different spots: proximally due to weakening of the supporting ligaments of the lowest section of the abdominal cavity floor and distally due to dilation of the natural cleft in the pelvic floor muscles. Both types of posterior wall collapse can occur entirely independently of each other.

Prolapses may cause unpleasant sensations in the abdomen and local physical discomfort. Conservative treatment of uterine and anterior vaginal wall prolapses is possible by means of introducing a supportive ring of pressary in the vagina.

A pessary is known by the name "Hodge-pessary" which is manufactured of rigid material such as porcelain, ebonite, a hard plastic material, etcetera. After insertion in the vagina it functions in such manner that the pessary itself lies on the pelvic floor muscles and is thus supported by them. Simultaneously it supports the uterus or anterior vaginal wall either directly through local pressure or indirectly by causing increased tensening of the supportive tissues involved.

As the pessary itself must lean on the pelvic floor muscles, it should be large enough to remain positioned on the cleft in the pelvic floor even with downwardly directed abdominal pressure. However, in case of prolapse, the muscles of the pelvic floor are often flaccid and thereby their cleft is relatively wide, so that then the pessary would descend with the pelvic floor or slip outwardly through the widened cleft. A relatively large pessary, which thereby and by its construction will be of a somewhat resilient nature, does not solve this problem because it would laterally strain the vaginal wall too much or, because of its flexibility, would be pushed out as easily as it is inserted.

If the pelvic floor muscles have not weakened and thereby the cleft is not too wide, a pessary of adequate width cannot be inserted, especially when also the vaginal opening is relatively narrow as this may happen in post-menopause due to involution.

If in case of a major prolapse and wide vaginal cavity a large-sized rigid pessary can be inserted, the uterus and the anterior vaginal wall may yet prolapse through the orifice of the ring.

The known pessary meets only those requirements which are demanded of the pessary by only a few particular individual prolapses, it being noted that the individual anatomical situation depends on five diverse variables, to wit: the individual build of the vagina and its surrounding tissue; the nature and the degree of the prolapse; the condition of the pelvic floor muscles and the width of the vaginal opening.

In US-A-4516570 there is disclosed a pessary produced in a closed ring form and comprising a core of a malleable metal wire and a flexible sheath formed from a biocompatible polymeric material covering said core. However, before being introduced into the vagina, the pessary is folded into a narrow configuration so that it has first and second free ends and an intermediate portion between said ends, whereas on insertion the pessary is formed into a non-closed curve wherein said intermediate portion has a semicircular shape.

DE-A-1900196 discloses a so-called resilient pessary which is formed by an annular endless coiled spring coated by silicon rubber or a plastic material. Thus by the coiled spring said ring has a resilient character so that by a deformation a tension is created in the ring which tends to push the ring back into its initial shape. Therefor a resilient pessary of this type is easily pressed out and gives only little support to the bladder neck.

The object of the invention is to provide a ring-shaped pessary that entirely meets with individual requirements in case of pessary treatment of a prolapse.

This object is attained in that in the pessary according to the invention the core has the shape of a spiral of non-resilient nature so that in addition the circumferential dimension of the ring can, to some extent, be increased or decreased by hand, after which the ring retains the ultimately given circumferential dimension.

In a ring-shaped pessary formed in this way by a specific choice of the gauge of the spirally wound wire and of the spiral diameter in proportion to the ring diameter, the ring can be of sufficient flexibility to enable molding by hand to a permanent shape and circumferential dimension, while the ring still offers sufficient resistance to deformation due to the pressure exerted by tissues.

The shape and circumferntial dimension of a pessary according to the invention can be perfectly adapted to the individual anatomy, both prior to and after insertion, so that the ring, after being inserted, supports both the neck of the bladder and the uterus but the ring itself is supported as little as possible by the pelvic floor muscles so that the effect of the ring depends much less on the quality of these muscles.

The shape into which the ring should be bend is determined by the anatomical proportions within the vaginal cavity, an impression of which may be obtained e.g. by an internal examination of the vagina.

Essentially all occuring prolapses can be optimally treated with the pessary according to the invention. Additionally only rings of a few different sizes need be kept available in order to meet the demand from many individual vaginal sizes.

The ring is preferably constructed of a core of stainless steel or similar material, encased in a flexible casing, preferably consisting of silicone rubber. Stainless steel is resistant to influences emanating from the human body and can be reshaped frequently without breaking, while silicone rubber is a flexible material with excellent tissuecompatible qualities.

Advantageously the ring, in its initial form as seen from top, has the contour of an egg-shaped oval, while from lateral view, as seen towards the longest dimension, it has the shape of a segment of a circle.

Accordingly constructed the ring, after insertion, can lie with the upwardly directed pointed end behind the cervix, pushing up the posterior vault, while the upwardly directed rounded end can lie behind the publis, from that particular spot pushing the vagina up in the region of the bladder neck, so that the inserted ring leans practically exclusively on the symphysis pubis and the retropubic place of attachment of the pelvic floor muscles and the ring itself is then not mainly supported by the pelvic floor muscles. Moreover, because of the pressure exerted from the abdominal cavity both on the uterus and the posterior vaginal wall and consequently on the more pointed end of the ring, the opposite end of the ring is not pushed toward the vaginal opening, but because of the bow-shape it is pushed further up behind the symphysis so that there is less chance of the ring to be pushed out, whereas in that both ends of the ring extend upwardly within the vagina, chances of hindrance during intercourse are minimal.

Advantageously the ring can be fitted with a diaphragm, also enabling the pessary to serve as contraceptive.

The casing can at the least be partially hollow, the cavity (2) being filled with a liquid or powdered substance consisting preferably of a medication which can be delivered by diffusion to the pessary-user.

Additionally the coating can consist partly of a polymer for gradual release of anti-microbial agents, contraceptives or substituting hormones.

The cavity(s) may also contain therapeutic or measuring apparatus for instance electrodes with energy-source for treatment of incontinence by way of muscle-stimulation, or for measuring the tensions of the vaginal or uterine muscles. A transmitter may be provided for wirelessly transmitting the measurements.

The pessary according to the invention may also serve to relieve incontinence in that a strip of an elastic material is arranged on the outside of the ring which strip is fixed along its edges whereas the space between the strip and the ring is in communication with means for pumping a fluid into said space so that the strip is expanded. By pumping air into said space e.g. by pushing in a balloon the strip is expanded so that the urethra is pressed together and closed off.

The invention is elucidated further on the basis of the embodiment shown in the drawing, in which

figure 1 gives a top view of a pessary according to the invention;

figure 2 shows a pessary in side view;

figure 3 is a cross section on a larger scale along line III-III in figure 1; and

figure 4 shows a pessary for relieving incontinence.

As shown by the figures, the pessary according to the invention comprises a ring 1 which as seen from top is formed like an egg-shaped oval, as a result of which the ring has a more pointed end 2 and a rounder end 3.

In side elevation as shown in figure 2 the ring is shaped like a segment of a circle with a radius r and an included angle of preferably $120°$.

As shown in figure 3, the ring consists of a spiral-shaped core 4 of stainless steel and a casing 5 of silicone rubber enclosing said core. The silicone rubber may be pigmented so as to offer an esthetic flesh-coloured aspect. The silicone rubber can moreover be impregnated with silicone oil, resulting in a lubricated outer surface.

In order to meet the large individual differences and proportions of the vaginal cavity, rings are available of varying nominal diameters R, for instance a series of rings which, starting with the smallest ring of R = 50 mm runs up to the largest ring of R = 90 mm, the width D increasing stepwise from 6 mm to 12 mm.

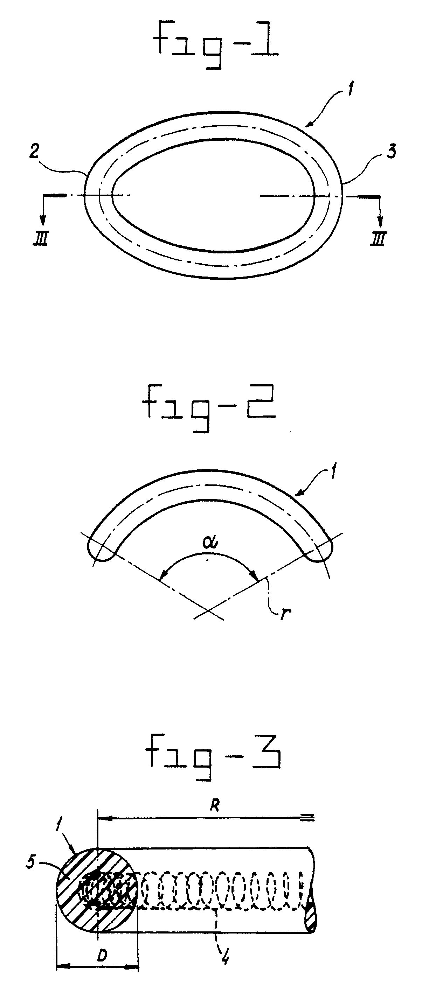

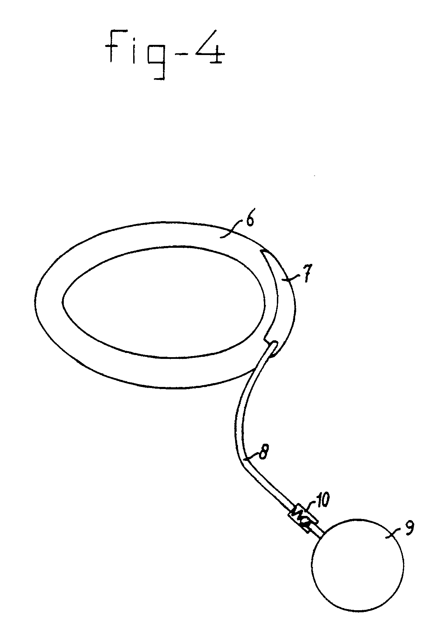

As shown by figure 4, a ring-shaped pessary 6 of the type illustrated in the figures 1-3 is provided with a strip 7 of an elastic material e.g. silicone rubber which is fixed along its circumferential edges to the outside of the ring so that a slit-shaped space is formed between the inner side of the strip 7 and the ring. Said space is in communication with a balloon 9 through tube 8 in which a non-return valve 10 is provided..

The pessary 6 is inserted in the vagina so that the strip 7 lies close to the urethra. By pressure on the balloon 9 a fluid is pumped into the slit-shaped space whereby the strip 7 is expanded outwardly and the urethra is pressed together and closed off. By operating the valve 10 the fluid is allowed to escape. Women who suffer from incontinence can regulate their urine release in this manner.

## Claims

1. Pessary particularly applicable in the treatment of prolapses of internal female sex organs, which pessary is formed by a ring-shaped member comprising a core of a malleable metal encased in a flexible casing, of which at least the outside consists of a biocompatible material such as a synthetic material, so that the member can be deformed by hand to any given shape after which the ring retains the ultimately given shape, characterized in that the core has the shape of a spiral of non-resilient nature, so that in addition the circumferential dimension of the ring-shaped member can be increased or decreased to some extent by hand after which the member retains the ultimately given circumferential dimension.

2. Pessary according to claim 1, characterized in that the ring, seen from top view, has the form of an egg-shaped oval, and seen from side view towards the longest dimension has the shape of a segment of a circle.

## Revendications

1. Pessaire qui trouve son application en particulier dans le traitement du prolapsus des organes génitaux féminins intérieurs, lequel pessaire est formé d'un élément en forme d'anneau comprenant un noyau en un métal malléable contenu dans une enveloppe flexible dont au moins l'extérieur est réalisé en un matériau biocompatible, tel qu'une matière plastique, de manière que l'élément peut être déformé à la main en toute forme donnée, après quoi, l'élément conserve la forme qui lui a été donnée en dernier lieu, caractérisé en ce que le noyau a la forme d'une spirale de nature non-résiliente, de manière que, en outre, la dimension circonférentielle de l'élément en forme d'anneau peut être augmentée ou diminuée, dans une certaine mesure, à la main, après quoi l'élément conserve la dimension circonférentielle qui lui a été donnée en dernier lieu.

2. Pessaire suivant la revendication 1, caractérisée en ce que la bague, vue de dessus, a la forme d'un ovale en forme d'oeuf et, vue de côté, vers la dimensions la plus longue, a la forme d'un segment de cercle.

## Patentansprüche

1. Pessar, insbesondere anwendbar bei der Behandlung von Vorfällen der inneren weiblichen Geschlechtsorgane, wobei das Pessar gebildet wird durch ein ringförmiges Element mit einem Kern aus verformbarem Metall, das in einer flexiblen Hülle eingeschlossen ist, von der mindestens die Außenseite aus einem bioverträglichen Material wie einem synthetischen Material besteht, so daß das Element von Hand in jede beliebige Form verformbar ist und der Ring die zuletzt gebildete Form beibehält, **dadurch gekennzeichnet,** daß der Kern die Form einer Spirale, die nicht elastisch ist, hat, so daß zusätzlich die Umfangsabmessung des ringförmigen Elements von Hand in gewissem Maße vergrößert oder verkleinert werden kann, wobei das Element die zuletzt gebildete Umfangsabmessung beibehält.

2. Pessar nach Anspruch 1, **dadurch gekennzeichnet,** daß der Ring von oben gesehen die Form eines eiförmigen Ovals aufweist und von der Seite in Richtung der längsten Abmessung gesehen, die Form eines Teilkreises aufweist.

# fig-1

# fig-2

# fig-3

Fig-4